# EUROPEAN PATENT APPLICATION

(11) **EP 1 314 418 A1**
(43) Date of publication of application: **28.05.2003**
(21) Application number: 02009053.6
(22) Date of filing: 23.04.2002
(51) Int. Cl.: A61K 7/13

(54) **Hairdye composition including 1-phenyl-3-methyl-5-pyrazolone and method for obtaining the same**

(30) Priority: 27.11.2001 KR 2001074275
(71) Applicant: Dong Sung pharmaceuticals Co., Ltd., Seoul (KR)
(72) Inventor: Jo, Bong Lim, Asan-Si, Chungcheongnam-Do (KR); Jo, Hyun Jin, Asan-Si, Chungcheongnam-Do (KR)
(74) Representative: Viering, Jentschura & Partner

(57) **Abstract**

A hairdye composition is disclosed, in which hair is normally dyed and at the same time the hairdye is maintained in a transparent state for a certain time period. A method for obtaining a hairdye composition containing phenylmethyl pyrazolone includes the steps of: dissolving fatty alcohol of 2∼25wt% based on total weight, surfactant of 0.5∼35wt% based on total weight, and phenylmethyl pyrazolone of 0.1∼30wt% based on total weight, at 70∼90°C; mixing the dissolved product with a dye of 0.1∼10wt% and purified water of 30∼55wt% based on total weight; cooling the mixture at 50∼60°C; and mixing an alkalizer of 2∼10wt% based on total weight with the resultant product.

## Description

### BACKGROUND OF THE INVENTION

### Field of the Invention

The present invention relates to a hairdye composition coloring a gray hair or decolored hair in a natural hair color or various colors, and more particularly to an oxidation hairdye composition having no color changes that may occur due to mixture of hairdye compositions in the course of hair coloring.

### Discussion of the Related Art

Generally, a hairdye is divided into three types in accordance with its conditions fixed to hair; a temporary hairdye, a semi-permanent hairdye, and a permanent hairdye.

The permanent hairdye is constructed in such a manner that a coloring agent is permeated into epidermis of hair to precipitate coloring molecules into hair cortex. Also, the permanent hairdye enables decoloring and coloring of hair by means of permeation and oxidation. Examples of the permanent hairdye include a vegetable dye, a metallic dye, a mixture dye, and an oxidation dye(synthetic dye). Of them, the oxidation dye is mostly used as the permanent hairdye. As examples of the oxidation dye, there are an aniline derived dye, a synthetic organic dye, and an amino tint.

The oxidation hairdye includes a first composition containing a dye and an alkalizer and a second composition containing oxygenated water. A mixture of the first composition and the second composition is applied to hair for hair coloring. The alkalizer contained in the first composition inflates hair shaft and assists the dye to be fixed into the hair cortex. Ammonia water and monoethanolamine are mainly used as the alkalizer. The oxygenated water contained in the second composition oxidizes melanin of hair to make a color of hair bright and polymerizes the oxidation dye contained in the first composition to produce a unique color of the dye.

If the first composition is mixed with the second composition to dye hair, a dyeing action of hair occurs and at the same time oxygenated water contained in the mixture is combined with oxygen in the air, thereby causing a color of the mixture to be changed to a dark color as time passes.

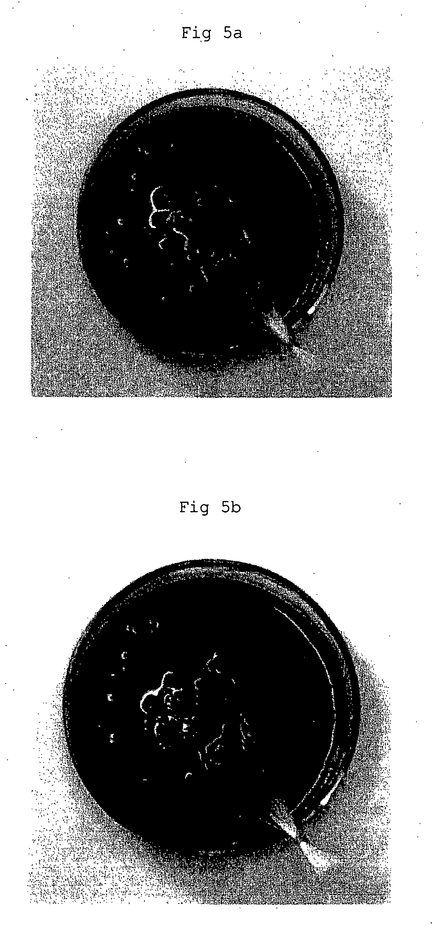

In other words, as shown in FIG. 5a, the mixture directly becomes dark as soon as the first composition is mixed with the second composition to dye hair. If hair is soaked in the mixture, the hair is colored as shown in FIG. 5b as time passes.

However, the conventional oxidation hairdye has several problems.

Since the hair coloring process cannot be checked in real time, a user arbitrarily determines a dye time period such as 10 minutes, 20 minutes, and 30 minutes. If the given time passes, the user washes the hairdye off the hair and then can check the state of hair coloring.

Furthermore, since a dyed color depends on thickness of hair, its state, and its damaged state, it is difficult to obtain a desired color unless the user exactly controls the dye time period.

Moreover, since the mixture of the conventional oxidation hairdye has a dark color, it may feel dislikable and dirty when it is washed off.

### SUMMARY OF THE INVENTION

Accordingly, the present invention is directed to a hairdye composition that substantially obviates one or more of the problems due to limitations and disadvantages of the related art.

An object of the present invention is to provide a hairdye composition having no color changes that may occur due to mixture of hairdye compositions, i.e., a first composition and a second composition.

Another object of the present invention is to provide a hairdye composition that can check a hair coloring process in real time using a transparent mixture of hairdye compositions.

Other object of the present invention is to provide a hairdye composition having a mixture of hairdye compositions that does not feel dislikable and dirty when it is washed off.

Additional features and advantages of the invention will be set forth in the description which follows, and in part will be apparent from the description, or may be learned by practice of the invention. The objectives and other advantages of the invention will be realized and attained by the scheme particularly pointed out in the written description and claims hereof as well as the appended drawings.

To achieve these and other advantages and in accordance with the purpose of the present invention, as embodied and broadly described, a hairdye composition according to the present invention includes a first composition containing a dye and an alkalizer, wherein the first composition includes phenylmethyl pyrazolone of 0.1∼30wt% based on total weight.

The first composition further includes at least one group selected from fatty alcohol, surfactant, fatty acid, and purified water.

The dye includes at least one group selected from oxidation dye intermediates such as P-phenylenediamine, P-aminophenol, O-aminophenol, resorcinol, toluene-2,5-diamine sulfate, and the alkalizer includes at least one group selected from strong ammonia water and monoethanolamine. The fatty alcohol includes at least one group selected from cetyl alcohol, stearyl alcohol, and behenyl alcohol. The surfactant includes at least one group selected from polyoxyethylene cetyl ether, and polyoxyethylene lauryl ether sodium sulfate. The fatty acid includes oleic acid.

The dye is composed of 0.1∼10wt%, the alkalizer of 2∼10wt%, the fatty alcohol of 2∼25wt%, the surfactant of 0.5∼35wt%, the fatty acid of 0.5∼20wt%, and the purified water of 30∼55wt%, based on total weight of the first composition.

In another aspect, a method for obtaining a hairdye composition containing phenylmethyl pyrazolone includes the steps of: dissolving fatty alcohol of 2∼25wt% based on total weight, surfactant of 0.5∼35wt% based on total weight, and phenylmethyl pyrazolone of 0.1∼30wt% based on total weight, at 70∼90°C; mixing the dissolved product with a dye of 0.1∼10wt% and purified water of 30∼55wt% based on total weight; cooling the mixture at 50∼60°C; and mixing an alkalizer of 2∼10wt% based on total weight with the resultant product.

It is to be understood that both the foregoing general description and the following detailed description are exemplary and explanatory and are intended to provide further explanation of the invention as claimed.

### BRIEF DESCRIPTION OF THE ATTACHED DRAWINGS

The invention will be described in detail with reference to the following drawings in which like reference numerals refer to like elements wherein:
FIG. 1 illustrates a chemical structure of phenylmethyl pyrazolone contained in a hairdye composition according to the present invention;
FIG. 2 is a block diagram illustrating processing steps according to the first embodiment of the present invention;
FIG. 3 is a block diagram illustrating processing steps according to the second embodiment of the present invention;
FIGS. 4a and 4b illustrate state changes after mixture of hairdye compositions according to the present invention; and
FIGS. 5a and 5b illustrate state changes after mixture of related art hairdye compositions.

### DETAILED DESCRIPTION OF THE PREFERRED EMBODIMENTS

Reference will now be made in detail to the preferred embodiments of the present invention, examples of which are illustrated in the accompanying drawings.

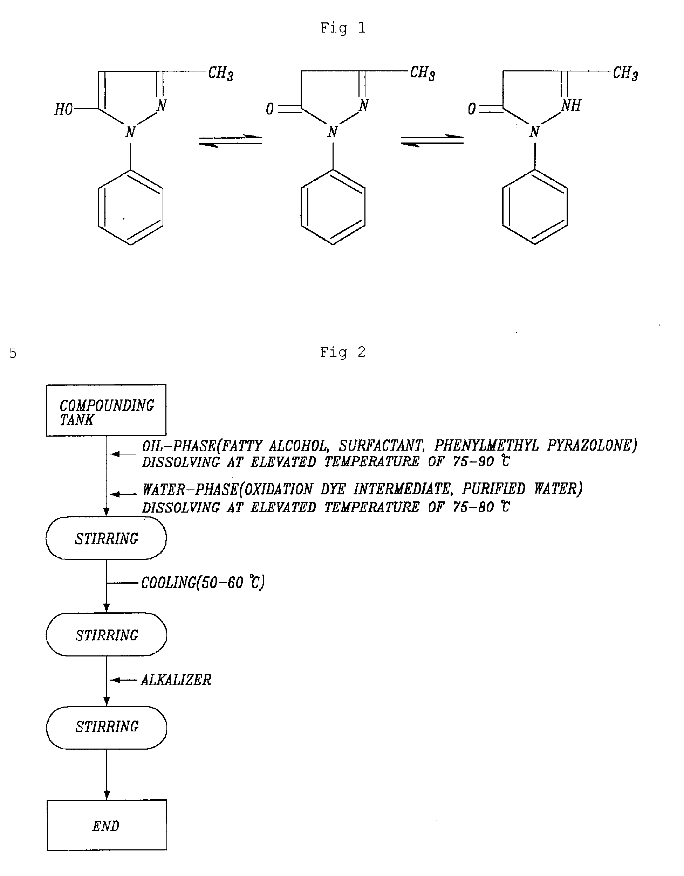

In the present invention, 1-phenyl-3-methyl-5 pyrazolone (phenylmethyl pyrazolone) is used as an anti-discoloration agent of a mixture and is derived from heterocyclicamine. Its chemical structure is as shown in FIG. 1.

The phenylmethyl pyrazolone is a crystalline powder ranging from white color to light brown color, and its boiling point is 287°C at 105mmHg and 191°C at 17mmHg.

The phenylmethyl pyrazolone was identified by experiment that when the first composition is mixed with a second composition containing oxygenated water, it acts so as not to oxidize the mixture in a black color due to oxygenated water and oxygen in the air.

The first composition of the hairdye according to the present invention and a method for obtaining the same will now be described with reference to the preferred examples.

### Example 1

As shown in FIG. 2, fatty alcohol composed of cetyl alcohol of 4.0wt%, stearyl alcohol of 3.0wt%, and behenyl alcohol of 7.0wt% based on total weight of the first composition, surfactant of 15.0wt% composed of polyoxyethylene cetyl ether based on total weight of the first composition, and phenylmethyl pyrazolone of 7.0wt% based on total weight of the first composition were dissolved at 75°C∼85°C.

Subsequently, the dissolved product was mixed with oxidation dye intermediates such as P-phenylenediamine of 2.4wt%, P-aminophenol of 0.6wt%, O-aminophenol of 0.1wt%, and resorcinol of 0.8wt%, and purified water of 54.1wt%. Then, the resultant product was dissolved at an elevated temperature of 75°C∼85°C.

After the dissolved product was mixed with the purified water, the mixture was cooled at 50°C∼55°C and mixed with monoethanolamine of 3.0wt% and strong ammonia water of 3.0wt% constituting alkalizer. Thus, a dye color base (first composition) was obtained in the form of cream.

A second composition, i.e., oxygenated water was added to the first composition obtained as above and hair to be dyed was let alone for 30 minutes.

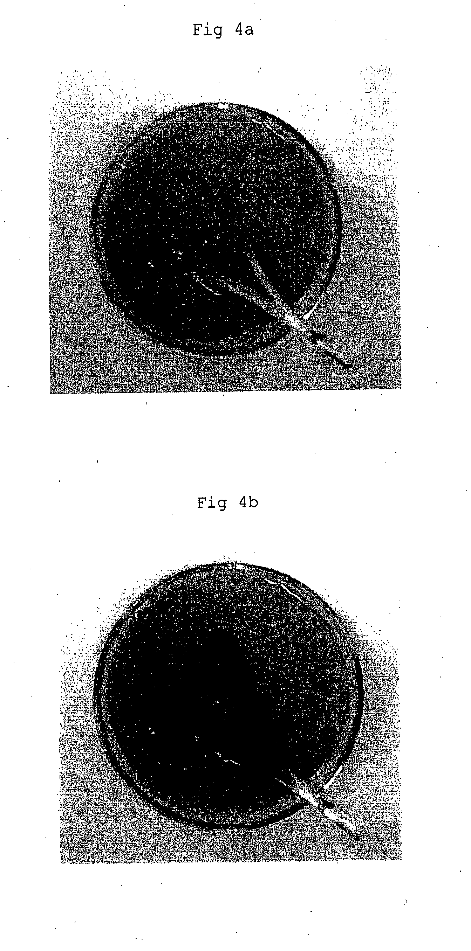

As a result, as shown in FIGS. 4a and 4b, no color changes of the hairdye occur even after the first composition has been mixed with the second composition or 30 minutes have elapsed. That is, it was identified by experiment that only hair was dyed in a state where the hairdye is transparent without any color exchanges.

### Example 2

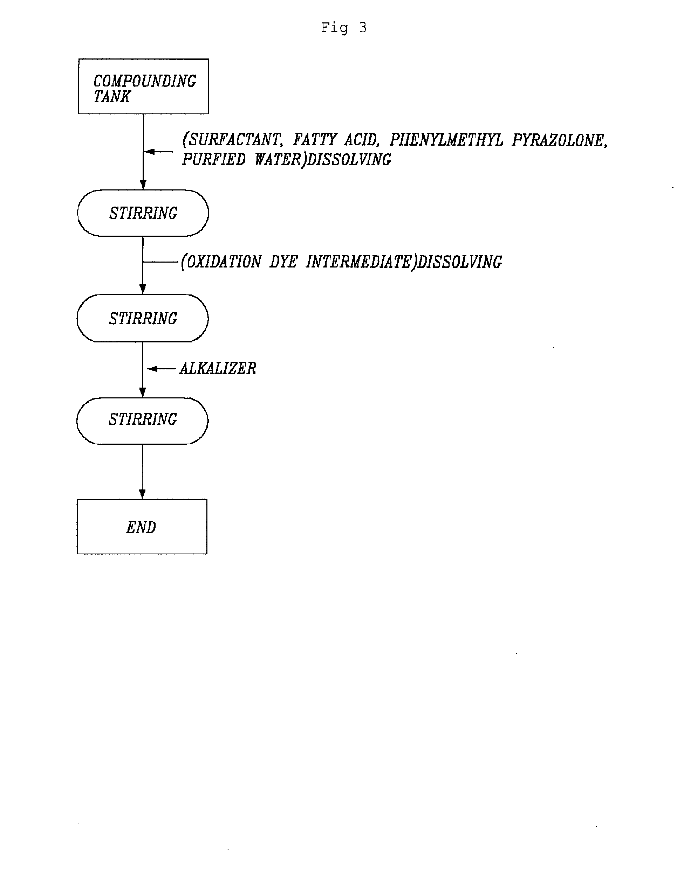

As shown in FIG. 3, based on total weight of the first composition, surfactant of 19.0wt% which is composed of polyoxyethylene lauryl ether sodium sulfate, oleic acid of 15.0wt% which is fatty acid, and phenylmethyl pyrazolone of 10.0wt% were mixed with purified water of 41.7wt% and stirred.

Subsequently, the stirred product was mixed with oxidation dye intermediates such as toluene-2,5-diamine sulfate of 6.0wt%, P-aminophenol of 0.8wt%, 0-aminophenol of 0.3wt%, and resorcinol of 1.2wt%, and then dissolved and stirred.

Afterwards, strong ammonia of 3.0wt% constituting alkalizer was mixed with monoethanolamine of 3.0wt%, so that a hairdye color base (first composition) was obtained in the form of cream.

A second composition, i.e., oxygenated water was added to the first composition obtained as above. Then, hair to be dyed was soaked in the mixture of the first composition and the second composition and was let alone for 30 minutes.

As a result, the same effect as that of the example 1 was obtained.

It was identified that in the oxidation hairdye according to the present invention containing phenylmethyl pyrazolone, as shown in FIGS. 4a and 4b, no color changes of the hairdye occurred when the first composition was mixed with the second composition and hair was dyed in a state where the hairdye is transparent without any color exchanges. That is, the hair coloring state was checked without washing the hairdye off.

A polymer structure of phenylmethyl pyrazolone, as shown in FIG. 1, forms a membrane between the oxidation dye and the oxygenated water in the mixture as a pentagonal ring substituent added to benzene ring changes to three reversible types, so as not to mix the oxidation dye with the oxygenated water. It is assumed that this causes no color changes in the hairdye.

As a result of the experiment, the more the content of phenylmethyl pyrazolone is, the longer the oxidation delay time is. It was noted that when phenylmethyl pyrazolone ranges from 0.6wt% to 200.0wt% based on total weight of the hairdye composition, it effectively delays oxidation of the mixture without affecting dye colors of hair.

Particularly, the hairdye of the first composition was obtained by mixing oxidation dye intermediates, i.e., the dye with phenylmethyl pyrazolone at a weight ratio of 1:1. If the second composition of oxygenated water was mixed with the first composition, oxidation delay effect occurred for about one hour or greater. Color changes of the hairdye slowly occurred after one hour. Since hair coloring is usually completed within 30 minutes and the hairdye is washed off directly after hair coloring, the oxidation delay of one hour or greater does not affect hair coloring according to the present invention.

As described above, the oxidation hairdye according to the present invention has the following advantages.

Since phenylmethyl pyrazolone is added to the hairdye composition in a predetermined content, hair is normally dyed and at the same time the hairdye is maintained in a transparent state for a certain time period. Therefore, the hair coloring process can be checked in real time and the hairdye can be washed off without feeling dislikable or dirty.

The foregoing embodiments are merely exemplary and are not to be construed as limiting the present invention. The present teachings can be readily applied to other types of apparatuses. The description of the present invention is intended to be illustrative, and not to limit the scope of the claims. Many alternatives, modifications, and variations will be apparent to those skilled in the art.

## Claims

1. A hairdye composition comprising a first composition containing an oxidation dye and an alkalizer and a second composition containing oxygenated water, wherein the first composition includes phenylmethyl pyrazolone of 0.1∼30wt%.

2. The hairdye composition as claimed in claim 1, wherein the first composition further includes at least one group selected from fatty alcohol, surfactant, fatty acid, and purified water.

3. The hairdye composition as claimed in claim 2, wherein the oxidation dye includes at least one group selected from P-phenylenediamine, P-aminophenol, O-aminophenol, resorcinol, and toluene-2, 5-diamine sulfate, the alkalizer includes at least one group selected from strong ammonia water and monoethanolamine, the fatty alcohol includes at least one group selected from cetyl alcohol, stearyl alcohol, and behenyl alcohol, the surfactant includes at least one group selected from polyoxyethylene cetyl ether and polyoxyethylene lauryl ether sodium sulfate, and the fatty acid includes oleic acid.

4. The hairdye composition as claimed in claim 2, wherein the first composition is obtained at a weight ratio of the oxidation dye of 0.1∼10wt%, the alkalizer of 2∼10wt%, the fatty alcohol of 2∼25wt%, the surfactant of 0.5∼35wt%, the fatty acid of 0.5∼20wt%, and the purified water of 30∼55wt%.

5. A method for obtaining a hairdye composition containing phenylmethyl pyrazolone includes the steps of:
dissolving fatty alcohol of 2∼25wt% based on total weight, surfactant of 0.5∼35wt% based on total weight, and phenylmethyl pyrazolone of 0.1∼30wt% based on total weight, at 70∼90°C;
mixing the dissolved product with a dye of 0.1∼10wt% and purified water of 30∼55wt% based on total weight;
cooling the mixture at 50∼60°C; and
mixing an alkalizer of 2∼10wt% based on total weight with the resultant product.
